(19) Europäisches Patentamt
European Patent Office
Office européen des brevets

(11) Publication number: **0 068 563**
**A2**

(12) **EUROPEAN PATENT APPLICATION**

(21) Application number: 82200744.9

(22) Date of filing: 16.06.82

(51) Int. Cl.³: **C 07 D 333/60**, C 07 D 209/18, C 07 D 235/16, C 07 D 307/79, C 07 D 277/64, C 07 D 279/20, C 07 D 265/38, C 07 D 311/82, C 07 D 335/12, C 07 D 333/76

(30) Priority: 23.06.81 US 276554

(43) Date of publication of application: 05.01.83 Bulletin 83/1

(84) Designated Contracting States: BE CH DE FR GB IT LI LU NL SE

(71) Applicant: THE PROCTER & GAMBLE COMPANY, 301 East Sixth Street, Cincinnati Ohio 45202 (US)

(72) Inventor: Fawzi, Mahdi Bakir, 5866 Red Oak Drive, Fairfield Ohio 45014 (US)

(74) Representative: Suslic, Lydia et al, Procter & Gamble European Technical Center Temselaan 100, B-1820 Strombeek-Bever (BE)

(54) **Heterocyclic acetic acid compounds and compositions for treating bone diseases.**

(57) Certain acetic acid derivatives of benzothiophene, benzimidazole, phenothiazine, benzofuran, dibenzothiophene, and similar heterocyclic compounds have been found to inhibit bone resorption and to enhance bone formation. The compounds of this invention are therefore useful in the treatment of diseases characterized by anomalous bone metabolism.

**EP 0 068 563 A2**

0068563

# HETEROCYCLIC ACETIC ACID COMPOUNDS AND COMPOSITIONS FOR TREATING BONE DISEASES

Mahdi B. Fawzi

## Technical Field

This invention relates to novel compounds capable of modifying the balance between bone production and bone resorption in warm-blooded animals, including humans. The invention further relates to a novel method for treating bone diseases characterized by imbalance between bone formation and bone resorption.

The condition in which hard bone tissue is lost disproportionately to the development of new hard tissue is generally referred to as osteoporosis. As a result of this imbalance, bone mass is reduced, marrow and bone spaces become, enlarged and skeletal integrity can be compromised to a point where there is bone pain and/or mechanical failure (i.e. fracture) upon normal demand. Osteoporosis can be subclassified as post-menopausal, senile, drug induced (e.g. by adrenocorticoid, as can occur in steroid therapy), disease induced (e.g. arthritic and tumor), etc. However, the manifestations are essentially the same.

As far as is known, no universally accepted medical treatment for osteoporosis has been developed prior to this invention. Although calcium supplementation, vitamin D and its derivatives, sodium fluoride, estrogens, calcitonin, anabolic steroids and polyphosphonates, either used alone or in combination, may provide some benefits, all these agents appear to involve serious drawbacks. The polyphosphonates are known to reduce anomalous mobilization of calcium phosphates in animals, thus reducing the resorption of bone tissue. Importantly, however, the polyphosphonates have only limited bone formation enhancement action, and some may even inhibit the formation of bone tissue if used at high levels. Hence, these compounds may successfully reduce the loss of bone mass, but do not restore the normal turnover of bone cells in cases where the rate of bone formation is abnormally low. Other compounds which have been reported to reduce the ratio bone resorption rate/bone formation rate are 2-thiophene

BAD ORIGINAL

carboxylic acid (I);

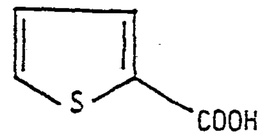

(I)

and compounds of the formula (II)

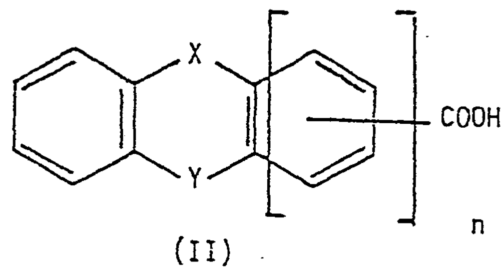

(II)

wherein Y = $(>C = O)_m$, n = 0 or 1, m = 0 or 1, X = S, O, or NH,

the carboxyl group must be on the 1, 2 or 3 position relative to X, and when X is NH and n = 0, the carboxyl group cannot be at the 2 position. From the published data it cannot be determined whether these compounds act <u>via</u> reduction of bone resorption, promotion of bone formation, or both. However, test data indicate that the compounds of formula (II) promote bone formation, but show only limited bone resorption inhibition. Treatment of osteoporosis with these compounds would therefore result in an excessively high bone cell turnover rate.

It has been discovered that the novel compounds of the present invention not only inhibit bone resorption, but also enhance bone formation. These novel compounds are therefore extremely suitable for use in the treatment of osteoporosis. It has further been discovered that known bone formation enhancers can be modified chemically so as to provide them with bone resorption inhibition properties as well.

BAD ORIGINAL

It is therefore an object of this invention to provide novel compounds which have utility in the treatment of conditions involving an anomalous bone resorption rate and/or an anomalous bone formation rate. It is a further object of this invention to provide compositions for the treatment of conditions involving an anomalous bone resorption rate and/or an anomalous bone formation rate. It is also an object of this invention to provide an improved method for treatment of such conditions.

## Background Art

The inhibition of spontaneous and parathyroid hormone induced bone resorption by 2-thiophene carboxylic acid and related compounds is discussed by Fang, et al. in Science, 172 (1971), 163-165. U.S. Patent No. 4,101,668 (1978) to Samour, et al. discloses the use of thionaphthene-2-carboxylic acid, thioxanthene-9-one-4-carboxylic acid, indol-2-carboxylic acid and their isomers in the treatment of osteoporosis.

Robin, et al., Calcif. Tissue Int. 33 (1981) 299, showed that 2-thiophene carboxylic acid does influence the calcium release from bone tissue, without influencing the cyclic AMP level.

## Detailed Description of the Invention

By "benzothiophene" herein is meant "benzo[B]thiophene."

By "benzofuran" herein is meant "benzo[B]furan".

The compounds of this invention have the formula

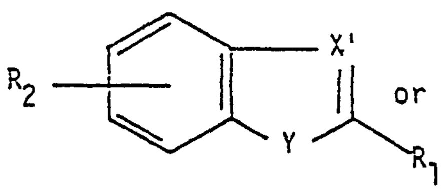

(III)

or

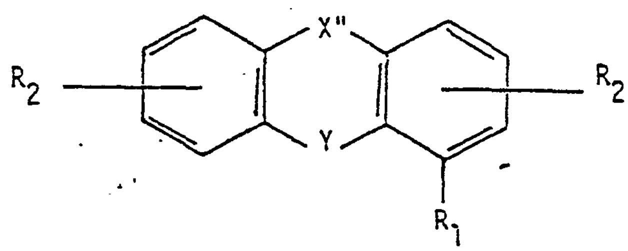

(IV)

or

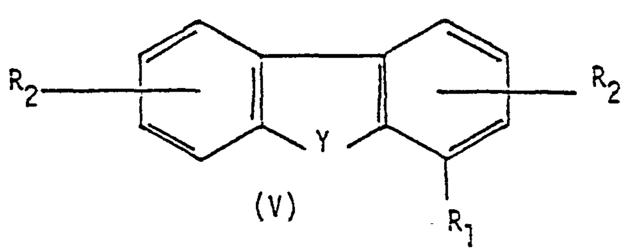

(V)

BAD ORIGINAL

wherein $R_1$ is - $CHR_2COOH$, and salts and esters thereof; $R_2$ is hydrogen, alkyl, alkoxy, - OH, - Cl, - F, - $NH_2$, - COOH, $PO(OH)_2$, or - $SO_2OH$, and esters and salts thereof; X' is N, CH, COH, or $COCH_3$; X" is NH, S, O, $CH_2$, CHOH, $CHOCH_3$ or C = O, Y is NH, S or O.

Operable compounds of the above formula (III) include:
benzothiophene-2-propionic acid,
4-hydroxybenzothiophene-2-acetic acid,
5-hydroxybenzothiophene-2-acetic acid,
6-hydroxybenzothiophene-2-acetic acid methyl ester,
4-chlorobenzothiophene-2-acetic acid,
5-chlorobenzothiophene-2-acetic acid,
6-chlorobenzothiophene-2-acetic acid,
7-chlorobenzothiophene-2-acetic acid,
5-fluorobenzothiophene-2-acetic acid ethyl ester,
6-aminobenzothiophene-2-acetic acid,
7-aminobenzothiophene-2-acetic acid,
4-carboxybenzothiophene-2-acetic acid sodium salt,
6-carboxybenzothiophene-2-acetic acid,
5-phosphonobenzothiophene-2-acetic acid,
6-sulfobenzothiophene-2-acetic acid,
3-hydroxybenzothiophene-2-acetic acid,
2-(3-hydroxy-2-benzothiophene)-propionic acid,
4-hydroxybenzothiophene-2-chloroacetic acid,
3-methoxybenzothiophene-2-acetic acid,
indole-2-acetic acid,
indole-2-propionic acid,
4-hydroxyindole-2-acetic acid,
5-chloroindole-2-acetic acid,
6-fluoroindole-2-acetic acid,
7-aminoindole-2-acetic acid isopropyl ester,
4-carboxyindole-2-acetic acid,
5-phosphonoindole-2-acetic acid,
6-sulfoindole-2-acetic acid,
benzofuran-2-acetic acid,
5-chlorobenzofuran-2-acetic acid,
6-fluorobenzofuran-2-acetic acid,
benzimidazole-2-acetic acid,

0068563

3-hydroxybenzimidazole-2-acetic acid,
4-hydroxybenzimidazole-2-acetic acid,
5-chlorobenzimidazole-2-acetic acid,
2-(6-fluoro-2-benzimidazole)-butyric acid,
6-methoxybenzimidazole-2-acetic acid,
benzothiazole-2-acetic acid,
2-(2-benzothiazole)-propionic acid,
benzothiazole-2-fluoroacetic acid,
5-methoxybenzothiazole-2-acetic acid,
6-chloro, 5-methoxybenzothiazole-2-acetic acid,
benzoxazole-2-acetic acid,
4-fluorobenzoxazole-2-acetic acid,
6-methylbenzoxazole-2-acetic acid and
5-aminobenzoxazole-2-acetic acid.
2-(4-hydroxy-2-benzoxazole)-pentanoic acid,

Operable compounds of the above formula (IV) include
phenothiazine-4-acetic acid,
2-methoxyphenothiazine-4-acetic acid,
3,8-dichlorophenothiazine-4-acetic acid,
phenothiazine-1-acetic acid,
phenothiazine-1-hydroxy acetic acid,
2-(1-phenothiazine)-propionic acid,
7-methylphenothiazine-1-acetic acid,
phenoxanthin-4-acetic acid,
phenoxanthin-1-acetic acid,
2-chlorophenoxanthin-4-acetic acid,
3,7-dihydroxyphenoxanthin-1-acetic acid,
phenoxazine-1-acetic acid,
phenoxazine-4-acetic acid,
3-aminophenoxazine-1-acetic acid,
3-chloro, 7-methylphenoxazine-4-acetic acid,
xanthene-1-acetic acid,
3-fluoroxanthene-1-acetic acid,
2,6-dimethylxanthene-1-acetic acid,
thioxanthene-1-acetic acid,
3,7-dimethoxythioxanthene-1-acetic acid,

BAD ORIGINAL

thianthrene-1-acetic acid,
3-phosphonothianthrene-1-acetic acid,
3,6-diisopropylthianthrene-1-acetic acid and
2-methoxy, 7-chlorothianthrene-1-acetic acid.

Operable compounds of the above formula (V) include
dibenzothiophene-4-acetic acid,
1,9-dimethyldibenzothiophene-4-acetic acid,
2-hydroxydibenzothiophene-4-acetic acid,
2,8-dichlorodibenzothiophene-4-acetic acid,
3-butoxydibenzothiophene-4-acetic acid,
dibenzofuran-4-acetic acid,
dibenzofuran-4-chloroacetic acid,
2-(4-dibenzofuran)-propionic acid,
2-hydroxydibenzofuran-4-acetic acid,
1-sulfodibenzofuran-4-acetic acid,
2-fluoro, 7-chlorodibenzofuran-4-acetic acid,
carbazole-4-acetic acid,
1-fluorocarbazole-4-acetic acid,
1-hydroxy, 9-methyl-carbazole-4-acetic acid and
2,8, di-t-butyl-carbazole-4-acetic acid.

Preferred compounds are those wherein $R_1$ is - $CH_2COOH$. Y is preferably NH or S. More preferred are the compounds wherein $R_2$ is hydrogen and Y is NH or S. Still more preferred are the compounds wherein $R_2$ is hydrogen, Y is NH or S, X' is N or CH, and X" is NH, S, or $CH_2$.

Most preferred compounds are benzothiophene-2-acetic acid (VI) and benzimidazole-2-acetic acid (VII).

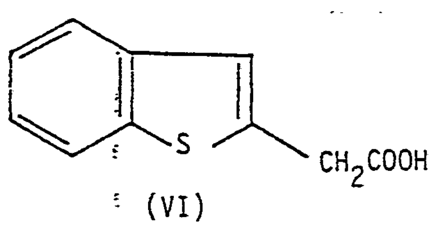

(VI)

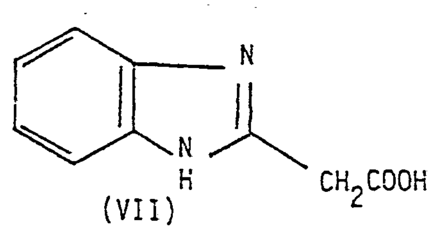

(VII)

The novel compounds of this invention are characterized by comprising a heterocyclic structure fused with one or two aromatic ring structures, and an acetic acid radical at the 2-position relative to the Y-atom. It has now been discovered that compounds having these structural characteristics have a highly beneficial effect on the metabolism of bone in warm-blooded animals. These compounds both enhance the formation of hard bone tissue and inhibit the resorption of hard bone tissue. The compounds known thus far in the treatment of conditions characterized by abnormal bone metabolism either only inhibit bone resorption, or only enhance bone formation. The novel compounds of this invention therefore provide a substantial improvement over presently available treatments of bone diseases.

It has further been discovered that certain compounds which are chemically related to the novel compounds of this invention are also useful in treating diseases characterized by abnormal bone metabolism. In particular, compounds of the formula (VIII):

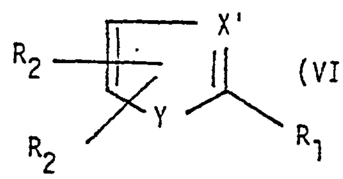

(VIII)

wherein $R_1$ is - COOH or - $CHR_2COOH$, and salts and esters thereof; $R_2$ is hydrogen, alkyl, alkoxy, - OH, - Cl, - F, - $NH_2$, - COOH, - $PO(OH)_2$, or - $SO_2OH$, and esters and salts thereof, provided that $R_1$ can only then be COOH when at least one $R_2$ is not hydrogen; X' is N, CH, COH, or $COCH_3$; Y is NH, S, or O, show significant bone metabolism regulating activity. Preferred compounds of formula (VIII) have $R_1$ is $CH_2COOH$ or a salt or an ester thereof; $R_2$ is preferably hydrogen; X' is preferably N or CH; Y is preferably NH or S.

Other compounds, which possess bone metabolism regulating properties are the compounds of the formula (III), (IV) or (V) wherein $R_1$ is - COOH or - $CHR_2COOH$, and salts and esters thereof; $R_2$ is hydrogen, alkyl, alkoxy, - OH, - Cl, - F, - $NH_2$, - COOH, - $PO(OH)_2$, or - $SO_2OH$, and esters and salts thereof provided that $R_1$ can only then be - COOH when at least one $R_2$ is

BAD ORIGINAL

not hydrogen; X' is N, CH, COH, or $COCH_3$; X" is NH, S, O, $CH_2$, CHOH, $CHOCH_3$ or C = O; Y is NH, S or O. Preferred compounds are these wherein X' is N or CH, X" is NH, S, or $CH_2$, and Y is NH or S. This class of compounds encompasses the novel compounds of this invention which have the highly desirable acetic acid substituent on the 2-position relative to the hetero atom. The class also encompasses compounds having the less desirable carboxylic acid substituent, the activity of which has been boosted by the presence of an electron donating substituent on the aromatic ring.

The compounds of formula (III), (IV) and (V) wherein $R_1$ is - $CH_2COOH$ can be synthesized from the corresponding carboxaldehydes (i.e. the corresponding compounds wherein $R_1$ is -CHO) using tetraethyl dimethylaminomethylene diphosphonate. Reaction of the carboxaldehyde with the diphosphonate gives the corresponding enamine phosphonate, which can be hydrolyzed in 12 N HCl to the acetic acid compound. The substituted derivatives can be prepared therefrom by standard organic synthesis techniques.

The compounds of this invention can also be prepared from the corresponding substituted benzothiophenes, indoles, benzofurans, benzimidazoles, benzothiazoles, benzoxazoles, phenothiazines, phenoxanthins, phenoxazines, xanthenes, thioxanthenes, thianthrenes, dibenzothiophenes, dibenzofurans, and carbazoles. The synthesis of these substituted heterocyclic compounds is disclosed in Rodd's Chemistry of Carbon Compounds, 2nd edition, S. Coffey, editor, Volume IV (Published by Elsevier, 1973), and references cited therein.

The substituted heterocyclic compound is converted to the acetyl derivative by a direct Friedel-Crafts acetylation. The acetyl derivative is subsequently heated with sulphur and a primary or secondary amine, e.g. methylamine or morpholine, and the resulting thioamide is hydrolyzed to form the required acetic acid derivative. The latter can, if desired, be converted to a pharmaceutically acceptable alkali metal or ammonium salt thereof by reaction with an appropriate base.

BAD ORIGINAL

## Example I

## Preparation of benzothiophene-2-acetic acid

Step 1

Tetraethyl dimethylaminomethylene diphosphonate was prepared as follows:

To a chilled solution (ice-bath of DMF (7.16g, 97.9mmol) in 150ml of dry $Et_2O$ was added dropwise with stirring a solution of oxalyl chloride (12.43g, 97.9mmol) in 20ml $Et_2O$. Following addition, the mixture was allowed to warm to room temperature and stirred for 1 hr. Triethyl phosphite (35.8g, 215mmol) was then added dropwise while stirring. After 1 hr the mixture was concentrated and distilled (125°, 0.07mm) to afford 29.4g (91% yield). The structure was confirmed with $^{31}P$ NMR and with $^{1}H$ NMR.

Step 2

Benzothiophene-2-carboxaldehyde was converted to the corresponding enamine phosphonate in the following manner.

To a suspension of NaH (0.40g, 16.7mmol) in 20ml of dry dioxane was added dropwise with stirring a solution of tetraethyl dimethylaminomethylene diphosphonate (5.52g, 16.7mmol) in 20ml dioxane. After 1 hr a solution of benzothiophene-2-carboxaldehyde (2.70g, 16.7mmol) in 20ml dioxane was added. The mixture was stirred at 50° for 1 hr, then concentrated. The residue was partitioned between $Et_2O$ and $H_2O$ and the aqueous layer was extracted with $Et_2O$ (3x). The combined organic extracts were dried ($MgSO_4$) and concentrated.

The residue was chromatographed on a column of silica gel (2:1 hexane : THF) to afford 3.93g (69% yield) of the product. $^{1}H$ NMR ($CDCl_3$, TMS) and 7.82-7.65 (m, 2H), 7.42-7.22 (m, 4H), 4.31-3.99 (m, 4H, $J$ = 7.3Hz), 2.69 (d, 6H, $J$ = 2.2Hz), 1.36 (t, 6H, $J$ = 7.1Hz); $^{31}P$ NMR ($CDCl_3$) 16.32 ppm downfield from 85% $H_3PO_4$ (ext.ref.); CI mass spectrum m/e 340 ($MH^+$).

0068563

Step 3

The product of Step 2 was hydrolized to benzothiophene-2-acetic acid as follows.

The enamine phosphonate (0.50g, 1.47mmol) in 50ml 12 N HCl was refluxed for 30 minutes. The mixture was then cooled, poured onto 200ml of ice-$H_2O$, and extracted with $Et_2O$ (3x). The combined organic extracts were dried ($MgSO_4$), clarified with activated charcoal, and concentrated to afford 0.23g (81% yield) of white crystalline benzothiophene-2-acetic acid.

The compound thus obtained is converted to the alkali metal or ammonium salt by reaction with an appropriate base.

In the same manner are prepared:
4-hydroxy-benzothiophene-2-acetic acid, 5-chlorobenzothiophene-2-acetic acid, 6-aminobenzothiophene-2-propionic acid, 7-fluorobenzothiophene-2-sodium acetate, 4-hydroxyindole-2-potassium acetate, 5-chloroindole-2-ammonium acetate, 6-aminoindole-2-propionic acid, 7-methoxyindole-2-acetic acid, 5-ethoxy-thiazole-butyric acid, 6-fluorothiazole-2-[2-ethoxy] acetic acid, 2-methoxy-phenothiazine-4-acetic acid, 7-chlorophenothiazine-1-acetic acid, 3,7-difluorophenoxanthin-1-acetic acid, 7-aminophenoxazine-1-acetic acid, 3-hydroxyphenoxazine-1-potassium acetate, xanthene-1-acetic acid, 3-fluoroxanthene-1-acetic acid, 3,7-dichlorothioxanthene-1-acetic acid, 2-aminodibenzothiophene-4-ammonium acetate, dibenzofuran-4-lithium acetate, 9-chlorocarbazole-4-acetic acid, and 1,9-dihydroxy carbazole-4-ammonium acetate.

Example II

In an alternative synthesis, benzimidazole-2-acetic acid was prepared by hydrolysis of 2-cyanomethylbenzimidazole.

In a 50 ml round bottom flask fitted with magnetic stirrer, reflux condensor and oil bath, was placed 20 ml $H_2O$, 1.2g NaOH and

3 ml EtOH. The mixture was stirred until homogeneous and to it was added 2-cyanomethylbenzimidazole (1.58g, .01 mole). The mixture was heated to reflux and held for about 2-1/2 hrs. Completeness of hydrolysis was tested by the lack of $NH_4OH$ evolution (pH paper). The reaction mixture appeared to be fairly homogeneous and was dark brown. The mixture was stirred to room temperature about 45 min. and adjusted to pH = 6.0 (pH meter). A small amount of precipitate occurred which was filtered off.

The filtrate was further acidified with glacial acetic acid, until the pH was $< 2$ (Hydrion paper), whereupon a heavy precipitate formed. The precipitate was filtered, triturated with MeOH, and dried under vacuum at room temperature to give about 1g of product.

The acetic acid compound is converted to the sodium salt by reaction with NaOH, to the potassium salt by reaction with KOH, and to the ammonium salt by reaction with $NH_4OH$.

In a similar manner are prepared 3-chloroimidazole-2-sodium acetate, 4-fluoroimidazole-2-potassium acetate, 7-hydroxyimidazole-2-acetic acid, 4,5-dimethoxyimidazole-2-acetic acid, 6-carboxyimidazole-2-acetic acid and 7-propylimidazole-2-acetic acid.

## Example III

Benzothiophene-2-acetic acid was converted to the methyl ester thereof as follows.

To a solution of benzothiophene-2-acetic acid (100mg, 0.52 mmol) and methanol (67 mg, 2.08 mmol) in 10 ml of dry methylene chloride was added dicyclohexylcarbodimide (118 mg, 0.57 mmol) and 10 mg of 4-dimethylaminopyridine. The mixture was stirred under argon for 2.5 hrs at room temperature and then filtered. The filtrate was washed with water (3x50 ml), 0.5N HCl (3x50ml), and then water again (3x50 ml). The organic extract was dried over anhydrous magnesium sulfate and then concentrated. The concentrate was chromatographed (19:1 hexane : THF, silica gel) to afford 80 mg (75% yield) of methyl benzothiophene-2-acetic acid methyl ester.

The following esters are prepared from the corresponding acids in a similar manner: 3-methoxybenzothiophene-2-ethyl acetate, 7-fluoroindole-2-propyl acetate, 4-aminobenzofuran-2-methyl acetate, phenothiazine-4-t butyl acetate, 3-methylphenoxazine-4-methyl acetate and 1-chlorocarbazole-4-ethyl acetate.

Example IV

The compound 2-(2-benzothiophene)-propionic acid was synthesized from benzothiophene as follows.

Step 1.

To a solution of n-butyllithium (100 ml of a 1.5N hexane solution) in 200 ml of dry ether was added at room temperature a solution of benzothiophene (20.0g, 0.15 mol) in 50 ml of ether. Upon completion of addition the mixture was refluxed for 1 hr, then chilled to -20°C and maintained at this temperature while a solution of dimethylacetamide in 50 ml ether was added dropwise. The mixture was then stirred at 0°C for 1 hr. Saturated $NH_4Cl$ solution (250 ml) was then added dropwise while the reaction mixture was maintained at -10 to -20°C. The ether layer was separated and washed with 1N HCl (1x200 ml) and then water (1x150 ml) before drying over anh. $MgSO_4$ and concentration. The concentrate was chromatographed (19:1 hexane : THF, silica gel) to afford 15.9g (60% yield) of 2-acetylbenzothiophene as light yellow crystals.

Step 2.

Potassium t-butoxide (2.55g, 22.7 mmol) was added to an ice-cold solution of tosylmethyl isocyanide (2.22g, 11.3 mmol) in 15 ml DME. After stirring at 0°C for 5 min., 0.5 ml of methanol was added followed by 2.00g (11.3 mmol) of 2-acetylbenzothiophene. The mixture was stirred for 18 hrs. at room temperature, then poured onto 300 ml $H_2O$ and extracted with ether (3x100 ml). The combined organic extracts were dried over anh. $MgSO_4$ and concentrated. The concentrate was chromatographed (9:1 hexane : THF, silica gel) to afford 0.75g (35% yield) of 2-(2-benzothiophene) propionitrile.

BAD ORIGINAL

Step 3.

2-(2-Benzothiophene)-propionitrile (0.72g, 3.84 mmol) was refluxed in 40 ml concd. HCl solution for 3 hrs. The solution was poured onto 250 ml of ice-water and extracted with ether (3x). The combined ether extracts were washed with 1N NaOH solution. The basic wash was then acidified with 1N HCl solution and extracted with ether (3x). The combined ether extracts were dried (anh. $MgSO_4$) and concentrated to afford 0.42g (53% yield) of 2-(2-benzothiophene)-propionic acid (m.p. 120-121°C).

In a similar manner indole is converted to 2-(2-indole)-propionic acid, benzofuran to 2-(2-benzofuran)-propionic acid, benzimidazole to 2-(2-benzimidazole)-propionic acid, benzothiazole to 2-(2-benzothiazol)-propionic acid, benzoxazole to 2-(2-benzoxazole)-propionic acid, phenothiazine to 2-(4-phenothiazine)-propionic acid, or to 2-(1-phenothiazine)-propionic acid, phenoxanthin to 2-(4-phenoxanthin)-propionic acid, or to 2-(1-phenoxanthin)-propionic acid, phenoxazine to 2-(1-phenoxazine)-propionic acid or to 2-(4-phenoxazine)-propionic acid, xanthene to 2-(1-xanthene)-propionic acid, thioxanthene to 2-(1-thioxanthene)-propionic acid, thianthrene to 2-(1-thianthrene)-propionic acid, dibenzothiophene to 2-(4-dibenzothiophene)-propionic acid, dibenzofuran to 2-(4-dibenzofuran)-propionic acid, and carbazole to 2-(4-carbazole)-propionic acid.

## Example V

5-Chlorobenzofuran is prepared according to the method disclosed by Newman, et al., J. Org. Chem., 30 (1965) 4126. 5-Chlorobenzofuran is converted to 5-Chlorobenzofuran-2-acetic acid using the method of Example I, and to 2-(5-Chlorobenzofuran-2)-propionic acid using the method of Example III.

In a similar manner are prepared 5-fluorobenzofuran-2-acetic acid and 2-(5-fluorobenzofuran-2)-propionic acid.

Example VI

Using the method of Example III whereby in step 1 dimethylacetamide is replaced with dimethylpropionamide, the following compounds are synthesized: 2-(2-benzothiophene)-butyric acid, 2-(6-fluoroindole-2)-butyric acid, 2-(4-hydroxybenzimidazole-2)-butyric acid, 2-(4-phenothiazine)-butyric acid and 2-(2-fluorocarbazole-4)-butyric acid.

Another aspect of this invention is a pharmaceutical composition in unit dosage form useful in treating diseases characterized by abnormal bone metabolism, comprising a safe and effective amount of a compound of the formula

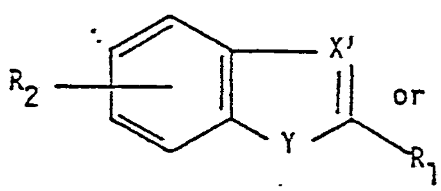

or

(III)

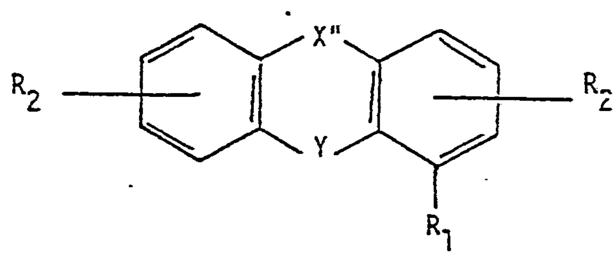

or

(IV)

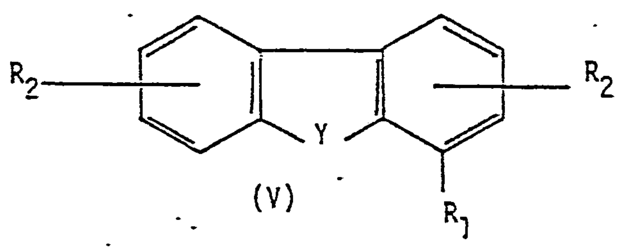

(V)

wherein $R_1$ is $CHR_2COOH$, and salts and esters thereof; $R_2$ is hydrogen, alkyl, alkoxy, $-OH$, $-Cl$, $-F$, $-NH_2$, $-COOH$, $-PO(OH)_2$, or $-SO_2OH$ and esters and salts thereof; X' is N, CH, COH, or $COCH_3$; X" is NH, S, O, $CH_2$, CHOH, $CHOCH_3$, or C=O; Y is NH, S, or O. The pharmaceutical composition may comprise any compound of formula (III), (IV) or (VI), or any mixture thereof. Preferred pharmaceutical compositions are those which comprise the preferred compounds of this invention.

Still another aspect of this invention is a method of treating diseases characterized by abnormal bone metabolism, comprising administering to a human or animal in need of such treatment a safe and effective amount of a compound having the formula:

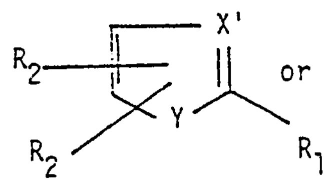

or

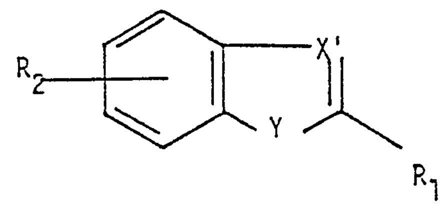

or

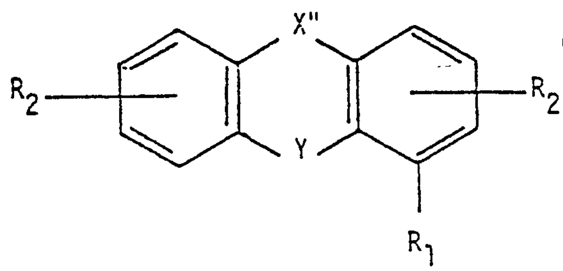

or

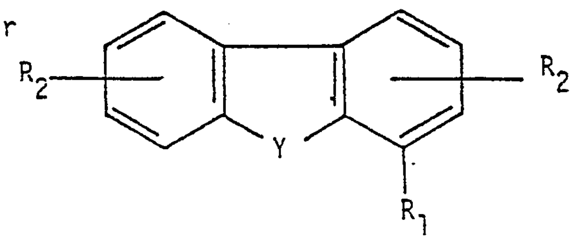

and mixtures thereof, wherein $R_1$ is - COOH or - $CHR_2COOH$, and salts and esters thereof; $R_2$ is hydrogen, alkyl, alkoxy, - OH, - Cl, - F, - $NH_2$, - COOH, - $PO(OH)_2$, or - $SO_2OH$, and esters and salts thereof; provided that $R_1$ can only then be - COOH when $R_2$ is not hydrogen; X' is N, CH, COH, $COCH_3$, or X" is NH, S, O, $CH_2$, CHOH, $CHOCH_3$ or C=O; Y is NH, S, or O; or mixtures of such compounds.

As used herein the term "safe and effective amount" denotes an amount which is sufficient to significantly change the bone metabolism of the diseased human or other animal, but low enough to avoid serious adverse side effects. Generally, the required dosage of heterocyclic acetic acid will vary with the particular condition being treated, the severity of the condition, the duration of treatment, and the specific heterocyclic acetic acid employed; however, single dosages can range from 0.01 to 500 mg per kilogram of body weight (hereafter "mg/kg"), preferrably 0.5 to 50 mg/kg, with up to four doses daily. The higher dosages in this range are, of course, required in the case of oral administration because of limited absorption. Dosages greater than about 500 mg/kg may produce toxic symptoms and should be avoided. Moreover, daily dosages greater than about 2,000 mg/kg are not required to produce the desired effect and may produce toxic side effects. Dosages of less than about 0.01 mg/kg do not materially affect abnormal bone metabolism, even administered intravenously.

BAD ORIGINAL

While it is not intended that this invention be limited by any particular theory, it is believed that the compounds of this invention affect the metabolism of bone cells by forming chelate complexes with cations which are involved in bone metabolism, like $Zn^{2+}$ and $Mn^{2+}$.

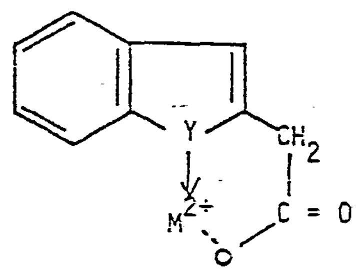

The position of the acetic acid radical at the 2-position with respect to the hetero-atom is critical for a sterically favorable position for the metal ion. The electronic structure of the heteroatom Y is favorable for charge transfer to the cation. The charge transfer is further favored by the presence of the aromatic ring and/or the electron donating substituent $R_2$. When the acetic acid radical is replaced with a carboxyl radical, the effectivity of the compound is significantly reduced. This effect is partially off-set if $R_2$ is a strong electron donating substituent.

## Animal Tests

The following test was used to establish <u>in vivo</u> periosteal and endosteal bone apposition rates and endosteal resorption rates. The test method is a modification of the method of Baylink, et al., J. Clin. Invest. 49, 1122-34 (1970), which is incorporated herein by reference.

## Animals and Treatment

Weanling male Sprague-Dawley derived rats weighing 50-60 grams (Charles River Breeding Labs, Wilmington, Massachusetts) were allowed 2-3 days to adapt to the laboratory environment before

BAD ORIGINAL

receiving any treatment. Throughout the adaptation and experimental periods the animals were allowed free access to tap water and food (Purina Lab Rat Chow, Ralston Purina Company, St. Louis, Missouri).

On the first day of the experiment all animals were given a subcutaneous injection of tetracycline hydrochloride at a dose of 20 mg/kg to label the skeleton. Test compound and saline (control animals) treatments were also started on the first day of the experiment. They were given subcutaneously under skin flap where the rear legs join the body using alternating sides each day. Since these animals were rapidly growing, body weights were taken daily and dosage adjusted accordingly.

Seven days after the animals received tetracycline they were given a subcutaneous injection of (2,4 bis)N-N'-di (carboxymethyl)amino methylfluorescein (DCAF) at a dose of 20 mg/kg to provide a second skeletal label.

Ten days after treatments were initiated, the animals were sacrificed by carbon dioxide asphyxiation and their left and right tibias and femurs taken and placed in 70% ethanol until they were further processed.

## Materials

The test compounds were given as solutions prepared in distilled water, and the pH was adjusted to 7.4. Solution concentrations were adjusted so that constant volumes of 0.2 ml/100 grams of body weight could be maintained for all animals.

Tetracycline hydrochloride (Tetracyn for intramusuclar injection, Pfizer Inc., New York, New York) was given as a 0.25% solution in saline. Dose volumes were 0.8 ml/100 grams of body weight.

DCAF, (2,4-bis)-N,N'di(carboxymethyl) amino methyl fluorescein, Nutritional Biochemicals Corporation, Cleveland, Ohio was given as a 1% solution in 2% sodium bicarbonate and 0.8% dextrose. This solution was filtered prior to administration to remove any undissolved particles. Dose volumes of the DCAF solution were 0.2 ml/100 grams of body weight.

BAD ORIGINAL

0068563

## Tissue Processing

The left tibiae were blocked stained with Villanueva's stain, embedded in methyl methacrylate and sectioned at 75-100 u. Cross sections were prepared from the shaft of the tibia where it is joined by the distal portion of the fibula.

## Bone apposition and resorption rates

These parameters were measured in tibial cross sections and in longitudinal sections through the proximal tibial epiphysis with a 5 mm micrometer which was superimposed optically over the section by placing it in the eyepiece of a microscope. Conversion of the values obtained to actual widths was established with a stage micrometer.

### a) Periosteal bone apposition rates

The width between fluorescent labels on the periosteal surface was established at 100x by making 4 measurements at 90 degree intervals around the circumference of the labeled area. Measurements were made from the middle of one label to the middle of the next. A mean value was then established for each surface and multiplied times the conversion factor to obtain the actual distance in microns. Bone apposition rates were then calculated by dividing this value by the number of days over which the fluorescent labels were given (i.e. 7 days).

b) Endosteal bone apposition rates

Endosteal bone apposition was established at 100x by making 4 measurements at equidistant points across the section from the bottom of the plate to the closest fluorescent label (the last label administered). A mean value was then established and multiplied times a conversion factor to obtain the actual distance in microns.

c) Bone resorption rates

The width from the closest periosteal label (pre-treatment label) to the resorbing surface on the endosteum was established at 100x by using a similar technique as mentioned above. A mean value was then established and multiplied times a conversion factor to obtain the actual distance in microns. Bone resorption rates were calculated by dividing this value by number of days over which the experiment was conducted (i.e. 10 days).

The following compounds were subjected to the above described animal test.

- dichloro-methylene-diphosphonate ($Cl_2MDP$)
- benzimidazole-2-acetic acid (BMZAA)
- benzothiophene-2-acetic acid (BTAA)
- benzothiophene-2-carboxylic acid (BTCA)

The test results are presented in the following table.

| Dose/Compd | | N[1)] | P BAR[2)] | EBAR[3)] | ERR[4)] |
|---|---|---|---|---|---|
| Control | | 3 | 14.7 | 4.0 | 3.6 |
| 16 uM | $Cl_2MDP$ | 3 | 14.8 | 3.2 | 1.3 |
| 80 uM | $Cl_2MDP$ | 4 | 14.8 | 3.1 | 0 |
| 5 uM | BMZAA[5)] | 5 | 15.5 | 3.8 | 1.0 |
| 16 uM | BMZAA[5)] | 5 | 18.1 | 3.4 | 1.5 |
| 5 uM | BTAA[5)] | 3 | 16.0 | 3.5 | 1.2 |
| 16 uM | BTAA[5)] | 3 | 18.1 | 3.9 | 0.9 |
| 5 uM | BTCA | 4 | 18.2 | 3.5 | 2.9 |
| 16 uM | BTCA | 5 | 16.2 | 3.4 | 3.3 |

1) number of animals
2) periosteal apposition rate (micron/day)
3) endosteal apposition rate (micron/day)
4) endosteal resorption rate (micron/day)
5) compound within the scope of the present invention

The data show that dichloromethylenediphosphonate does not enhance bone formation, and that benzothiophene-2-carboxylic acid does not significantly reduce bone resorption. The compounds within the scope of this invention reduce bone resorption as effectively as dichloromethylenediphosphonate, and enhance bone formation as effectively as benzothiophene-2-carboxylic acid.

In vitro bone resorption

The test used for the determination of in vitro bone resorption inhibition is described in detail by Raisz, et al., Endocrinology, 85 (1969) 446-52, incorporated herein by reference. Fetal rat bone was cultured in the medium described in this reference. Bone resorption was stimulated by addition of prostaglandin, $PGE_2$, and measured by the release of previously incorporated $^{45}Ca$ into the medium and by changes in the total bone calcium content. The effect of the tested compounds on bone resorption was compared to control cultures, and expressed as percent inhibition of bone resorption, defined as:

$$\frac{\text{bone resorption control} - \text{bone resorption test compound}}{\text{bone resorption control}} \times 100\%$$

0068563

PGE$_2$ Stimulated Bone Resorption in the Fetal Long Bone System

| Compound | Concentration ug/ml | Concentration Mole | % Inhibition of Bone Resorption |
|---|---|---|---|
| Thiophene-2-Carboxylic Acid | 50 ug/ml | 0.39 mM | 43 % |
| | 200 ug/ml | 1.56 mM | 47.8% |
| 5-Methoxyindole-2-Carboxylic Acid[1)] | 74.6 ug/ml | 0.39 mM | 47.5% |
| | 298.4 ug/ml | 1.56 mM | 80.1% |
| Thiophene-2-Acetic Acid[1)] | 55.47 ug/ml | 0.39 mM | 47.8% |
| | 221.9 ug/ml | 1.56 mM | 106.7% |

1) Compound within the scope of the present invention

The data in the table indicate that the *in vitro* bone resorption inhibition can be markedly improved by replacing carboxylic acid with acetic acid, or by incorporation of an aromatic ring and an electron donating substituent in the molecule.

## Example VII

Capsules are prepared by conventional methods, comprised as follows:

| Ingredient | mg per capsule |
|---|---|
| Benzothiophene-2-acetic acid | 350.00 |
| Starch | 55.60 |
| Sodium lauryl sulfate | 2.90 |

The above capsules administered orally twice daily substantially reduce bone decalcification and enhance bone formation in a patient weighing approximately 70 kilograms afflicted with osteoporosis. Similar results are attained when benzothiophene-2-acetic acid in the above described capsules is replaced with benzimidazole-2-acetic acid, benzofuran-2-acetic acid,

 0068563

benzothiazole-2-acetic acid, phenoxizine-4-acetic acid or dibenzothiophene-4-acetic acid.

Example VIII

Tablets are prepared by conventional methods, formulated as follows:

| Ingredient | mg per Tablet |
|---|---|
| Benzimidazole-2-acetic acid | 25.00 |
| Lactose | 40.00 |
| Starch | 2.50 |
| Magnesium stearate | 1.00 |

When administered orally four times daily, the above composition significantly enhances the turnover of bone tissue in a patient weighing approximately 50 kilograms, having a predisposition to low bone metabolism rates.

Similar results are achieved with tablets formulated as above but replacing benzimidazole-2-acetic acid with benzothiophene-2-acetic acid, indole-2-acetic acid, benzoxazole-2-acetic acid, phenothiazine-4-acetic acid, phenoxazine-1-acetic acid, xanthene-1-acetic acid, thianthrene-1-acetic acid, and carbazole-4-acetic acid, respectively.

Solutions for parenteral administration are prepared by dissolving the following acetic acid compounds in distilled water, adjusting the pH to 7.4 with sodium hydroxide and sterilizing the solution by standard sterilization techniques.

| Examples | Acetic Acid Compound | Conc. - mg/ml |
|---|---|---|
| IX | benzothiophene-2-acetic acid | 10.0 |
| X | benzimidazole-2-acetic acid | 15.0 |
| XI | 3-fluoroxanthene-1-acetic acid | 5.0 |
| XII | 1-hydroxycarbazole-4-acetic acid | 13.0 |
| XIII | 4,5-dimethoxy benzothiophene -2-acetic acid | 8.0 |
| XIV | 4-chlorofuran-2-acetic acid | 12.5 |

## CLAIMS

1. Compounds characterized in that they have the formula:

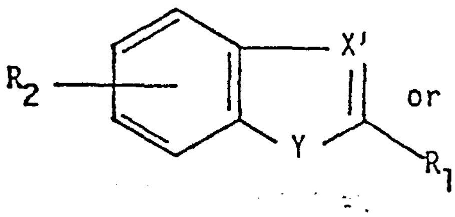

or

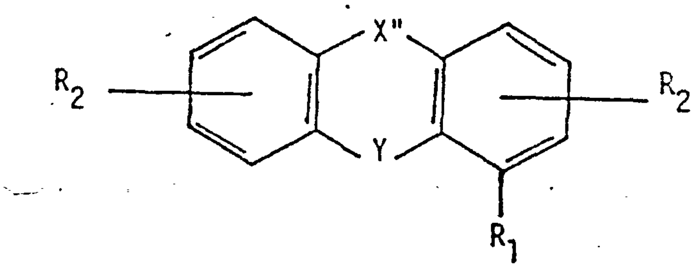

or

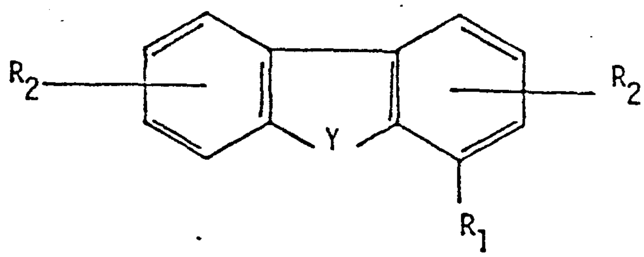

wherein $R_1$ is $-CHR_2COOH$, and salts and esters thereof; $R_2$ is hydrogen, alkyl, alkoxy, $-OH$, $-Cl$, $-F$, $-NH_2$, $-COOH$, $-PO(OH)_2$, or $-SO_2OH$, and esters and salts thereof, X' is N, CH, COH, or $COCH_3$; X" is NH, S, O, $CH_2$, CHOH, $CHOCH_3$, or C = O; Y is NH, S, or O.

2. The compounds of Claim 1 characterized in that $R_1$ is $-CH_2COOH$.

3. The compounds of Claim 1 or 2 characterized in that $R_2$ is hydrogen.

4. The compounds of any one of Claims 1-3 characterized in that X' is N or CH, and X" is NH, S or $CH_2$.

5. The compound characterized in that it has the formula:

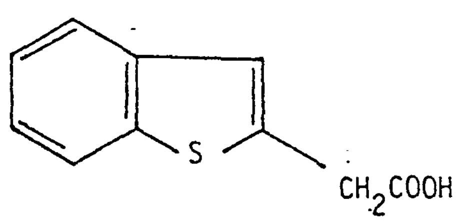

6. The compound characterized in that it has the formula:

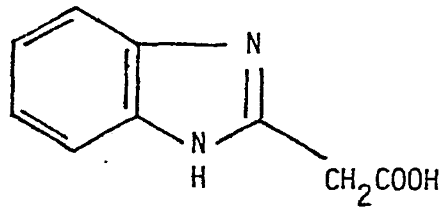

7. A pharmaceutical composition in unit dosage form useful in treating diseases characterized by abnormal bone metabolism, said composition being characterized in that it comprises a safe and effective amount of a compound of the formula:

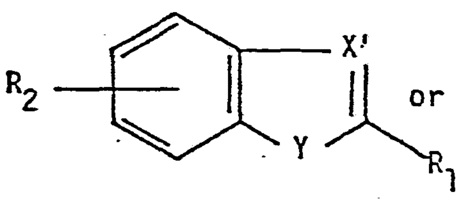

or

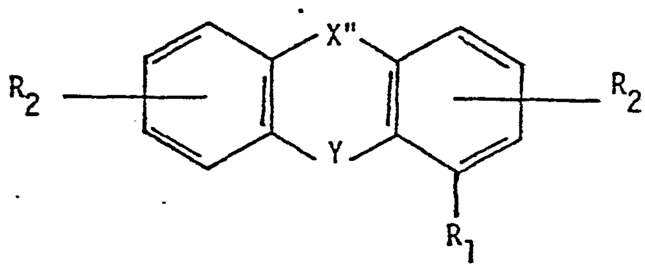

or

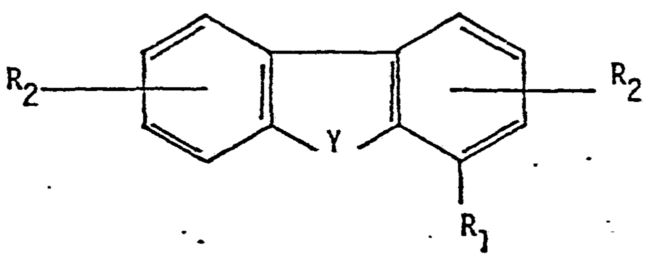

wherein $R_1$ is $- CHR_2 COOH$, and salts and esters thereof; $R_2$ is hydrogen, alkyl, alkoxy, - OH, - Cl, - F, $- NH_2$, - COOH, - $PO(OH)_2$, or $- SO_2OH$, and esters and salts thereof; X' is N, CH, COH, or $COCH_3$, X" is NH, S, O, $CH_2$, CHOH, $CHOCH_3$, or C = O; Y is NH, S, or O; or mixtures of such compounds.

8. The composition of Claim 7 characterized in that $R_1$ is - $CH_2COOH$ or a salt or ester thereof.

9. The composition of Claim 7 or 8, characterized in that $R_2$ is hydrogen.

0068563

10. The composition of any one of Claims 7-9 characterized in that X' is N or CH and X" is NH, S or $CH_2$.

11. A pharmaceutical composition in unit dosage form useful in treating diseases characterized by abnormal bone metabolism, said composition being characterized in that it comprises a safe and effective amount of the compound of the formula:

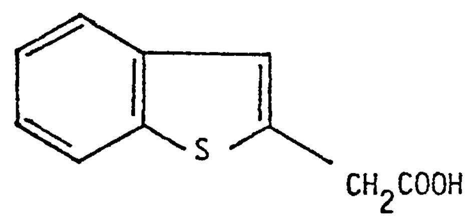

12. A pharmaceutical composition in unit dosage form useful in treating bone diseases characterized by abnormal bone metabolism, said composition being characterized in that it comprises a safe and effective amount of the compound of the formula: